(19) Europäisches Patentamt
European Patent Office
Office européen des brevets

(11) EP 0 753 517 A2

(12) **EUROPÄISCHE PATENTANMELDUNG**

(43) Veröffentlichungstag:
**15.01.1997 Patentblatt 1997/03**

(21) Anmeldenummer: **96110593.9**

(22) Anmeldetag: **01.07.1996**

(51) Int. Cl.$^6$: **C07D 471/04**, A61K 31/44
// (C07D471/04, 221:00, 209:00)

(84) Benannte Vertragsstaaten:
**AT BE CH DE DK ES FR GB GR IE IT LI LU MC NL PT SE**

(30) Priorität: **10.07.1995 DE 19525028**

(71) Anmelder: **BAYER AG**
**51368 Leverkusen (DE)**

(72) Erfinder:
- **Müller, Ulrich, Dr.**
  **42111 Wuppertal (DE)**
- **Connell, Richard, Dr.**
  **Trumbull, CT 06611 (US)**
- **Bischoff, Hilmar, Dr.**
  **42113 Wuppertal (DE)**
- **Denzer, Dirk, Dr.**
  **42115 Wuppertal (DE)**
- **Lohmer, Stefan, Dr.**
  **20133 Milano (IT)**
- **Wohlfeil, Stefan, Dr.**
  **40721 Hilden (DE)**
- **Grützmann, Rudi, Dr.**
  **42657 Solingen (DE)**

(54) **Amide und Sulfonamide von heterocyclisch substituierten Benzylaminen**

(57) Amide und Sulfonamide von heterocyclisch substituierten Benzylaminen werden hergestellt, indem man die entsprechenden Carbonsäuren zunächst in die korrespondierenden Amine überführt und diese anschließend am Aminstickstoff entsprechend substituiert. Die neuen Amide und Sulfonamide von heterocyclisch substituierten Benzylaminen können als Wirkstoffe in Arzneimitteln, insbesondere in antiatherosklerotischen Arzneimitteln verwendet werden.

EP 0 753 517 A2

## Beschreibung

Die vorliegende Erfindung betrifft Amide und Sulfonamide von heterocyclisch substituierten Benzylaminen, Verfahren zu ihrer Herstellung und ihre Verwendung als Arzneimittel, insbesondere als antiatherosklerotische Arzneimittel.

Es ist bekannt, daß erhöhte Blutspiegel von Triglyceriden (Hypertriglyceridämie) und Cholesterin (Hypercholestennämie) mit der Genese von atherosklerotischen Gefäßwandveränderungen und koronaren Herzkrankheiten assoziiert sind.

Ein deutlich erhöhtes Risiko für die Entwicklung koronarer Herzerkrankungen liegt darüberhinaus vor, wenn diese beiden Risikofaktoren kombiniert auftreten, was wiederum mit einer Überproduktion an Apolipoprotein B-100 einhergeht. Es ist daher nach wie vor ein starkes Bedürfnis, wirksame Arzneimittel zur Bekämpfung der Atherosklerose sowie koronarer Herzkrankheiten zur Verfügung zu stellen.

Die vorliegende Erfindung betrifft Amide und Sulfonamide von heterocyclisch substituierten Benzylaminen der allgemeinen Formel (I)

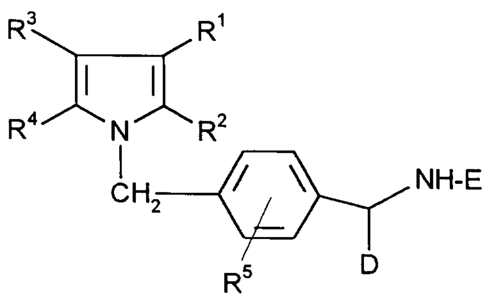

(I)

in welcher

$R^1$ und $R^2$ unter Einbezug der sie verbindenden Doppelbindung gemeinsam einen Phenyl- oder Pyridylring bilden,

$R^3$ und $R^4$ unter Einbezug der sie verbindenden Doppelbindung gemeinsam einen Phenylring oder einen 4- bis 8-gliedrigen Cycloalken-Rest bilden,
wobei alle unter $R^1$/$R^2$ und $R^3$/$R^4$ aufgeführten Ringsysteme gegebenenfalls bis zu 3-fach gleich oder verschieden durch Halogen, Trifluormethyl, Carboxy, Hydroxy, geradkettiges oder verzweigtes Alkoxy oder Alkoxycarbonyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy oder durch geradkettiges oder verzweigtes Alkoxy mit bis zu 4 Kohlenstoffatomen substituiert sein kann,

$R^5$ für Wasserstoff, Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano oder Carboxy steht,

D für Wasserstoff, Cycloalkyl mit 4 bis 8 Kohlenstoffatomen oder
für geradkettiges oder verzweigtes Alkyl mit bis zu 8 Kohlenstoffatomen steht,

E für Wasserstoff oder für einen Rest der Formel
$-CO-NR^6R^7$,

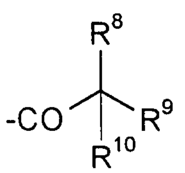

, L-$R^{11}$ oder

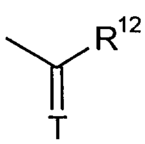

steht,

in welchen

$R^6$ und $R^7$ gleich oder verschieden sind und Wasserstoff, Phenyl oder geradkettiges oder verzweigtes Alkyl mit bis zu 6 Kohlenstoffatomen bedeuten,

$R^8$ Wasserstoff, geradkettiges oder verzweigtes Alkyl mit bis zu 6 Kohlenstoffatomen, Phenyl oder einen 5- bis 7-gliedrigen gesättigten oder ungesättigten Heterocyclus mit bis zu 3 Heteroatomen aus der Reihe S, N und/oder O bedeutet,
wobei die Cyclen gegebenenfalls bis zu 3-fach gleich oder verschieden durch Nitro, Carboxyl, Halogen, Cyano oder durch geradkettiges oder verzweigtes Alkoxycarbonyl mit bis zu 6 Kohlenstoffatomen oder durch geradkettiges oder verzweigtes Alkyl mit bis zu 6 Kohlenstoffatomen substituiert sind, das gegebenenfalls durch Hydroxy, Carboxyl oder durch geradkettiges oder verzweigtes Alkoxy oder Alkoxycarbonyl mit jeweils bis zu 6 Kohlenstoffatomen substituiert ist, und/oder die Cyclen gegebenenfalls durch eine Gruppe der Formel $-OR^{13}$ substituiert sind,
worin

$R^{13}$ für Wasserstoff oder geradkettiges oder verzweigtes Alkyl oder Alkenyl mit jeweils bis zu 6 Kohlenstoffatomen steht,

$R^9$ Carboxyl, Hydroxy, geradkettiges oder verzweigtes Alkoxycarbonyl mit bis zu 6 Kohlenstoffatomen oder eine Gruppe der Formel $-CH_2OH-$ oder $-O-CO-R^{14}$ bedeutet,
worin

$R^{14}$ für geradkettiges oder verzweigtes Alkyl mit bis zu 6 Kohlenstoffatomen steht,

$R^{10}$ Wasserstoff bedeutet,
oder

$R^9$ und $R^{10}$ gemeinsam einen Rest der Formel =O oder $=N-R^{15}$ bilden,
worin

$R^{15}$ für Hydroxy, geradkettiges oder verzweigtes Alkoxy mit bis zu 6 Kohlenstoffatomen oder einen Rest der Formel $-NH-CO-NH_2$ oder $-NH-CS-NH_2$ steht,

L eine Gruppe $-SO_2-$ oder -CO- bedeutet,

$R^{11}$ Phenyl oder einen 5- bis 7-gliedrigen aromatischen Heterocyclus mit bis zu 3 Heteroatomen aus der Reihe S, N und/oder O bedeuten, oder
geradkettiges oder verzweigtes Alkyl mit bis zu 6 Kohlenstoffatomen bedeutet, das gegebenenfalls durch Phenyl oder durch einen 5- bis 7-gliedrigen aromatischen Heterocyclus mit bis zu 3 Heteroatomen aus der Reihe S, N und/oder O substituiert ist, wobei alle Ringsysteme gegebenenfalls bis zu 3-fach gleich oder verschieden durch Hydroxy, Phenoxy, Halogen oder durch geradkettiges oder verzweigtes Alkyl oder Alkoxy mit jeweils bis zu 5 Kohlenstoffatomen substituiert sind,

T ein Sauerstoff- oder Schwefelatom bedeutet,

$R^{12}$ Phenoxy, Phenylthio oder geradkettiges oder verzweigtes Alkoxy mit bis zu 6 Kohlenstoffatomen bedeutet,

und deren Salze.

Die erfindungsgemäßen Amide und Sulfonamide von heterocyclisch substituierten Benzylaminen können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure,

Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di-bzw. Triethylamin, Di-bzw. Triethanolamin, Dicyclohexylamin, Dimethylaminoethanol, Arginin, Lysin, Ethylendiamin oder 2-Phenylethylamin.

Der cyclische-Rest ($R^3/R^4$) steht unter Einbezug der Doppelbindung des Grundgerüstes im Rahmen der Erfindung im allgemeinen für einen 5- bis 8-gliedrigen, vorzugsweise 5- bis 7-gliedrigen Kohlenwasserstoffrest wie beispielsweise für einen Phenyl-, Cyclopenten-, Cyclohexen-, Cyclohepten- oder Cycloocten-Rest. Bevorzugt sind der Cyclopenten-, Cyclohexen-, Cyclohepten- und der Phenyl-Rest.

Heterocyclus steht im Rahmen der Erfindung im allgemeinen für einen gesättigten oder ungesättigten 5- bis 7-gliedrigen, vorzugsweise 5- bis 6-gliedrigen Heterocyclus der bis zu 3 Heteroatome aus der Reihe S, N und/oder O enthalten kann. Beispielsweise seien genannt: Pyridyl, Thienyl, Furyl, Pyrrolyl, Thiazolyl, Oxazolyl, Imidazolyl, Morpholinyl oder Piperidyl. Bevorzugt sind Pyridyl und Thienyl.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Diese Mischungen der Enantiomeren und Diastereomeren lassen sich in bekannter Weise in die stereoisomer einheitlichen Bestandteile trennen.

Die erfindungsgemäßen Verbindungen unterliegen im Fall, daß E für einen Rest der Formel =N- steht, auch der E/Z-Isomerie. Sie können die E- oder Z-Konfiguration besitzen oder als E/Z-Gemisch vorliegen.

Bevorzugt sind Verbindungen der allgemeinen Formel (I),
in welcher

$R^1$ und $R^2$ unter Einbezug der sie verbindenden Doppelbindung gemeinsam einen Phenyl- oder Pyridylring bilden,

$R^3$ und $R^4$ unter Einbezug der sie verbindenden Doppelbindung gemeinsam einen Phenylring oder einen Cyclopenten-, Cyclohexen-, Cyclohepten-, Cycloocten-Rest bilden,
wobei alle unter $R^1/R^2$ und $R^3/R^4$ aufgeführten Ringsysteme gegebenenfalls bis zu 2-fach gleich oder verschieden durch Fluor, Chlor, Brom, Trifluormethyl, Carboxy, Hydroxy, geradkettiges oder verzweigtes Alkoxy oder Alkoxycarbonyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy oder durch geradkettiges oder verzweigtes Alkoxy mit bis zu 3 Kohlenstoffatomen substituiert sein kann,

$R^5$ für Wasserstoff, Fluor, Chlor, Brom, Trifluormethyl, Carboxy oder geradkettiges oder verzweigtes Alkyl, Alkoxy oder Alkoxycarbonyl mit jeweils bis zu 4 Kohlenstoffatomen steht,

D für Wasserstoff, Cyclobutyl, Cyclopentyl, Cyclohexyl, Cycloheptyl, Cylcooctyl oder für geradkettiges oder verzweigtes Alkyl mit bis zu 7 Kohlenstoffatomen steht,

E für Wasserstoff oder für einen Rest der Formel
$-CO-NR^6R^7$,

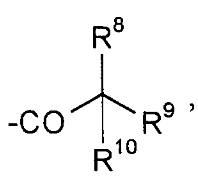
, $L-R^{11}$ oder
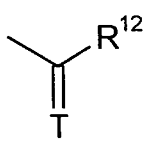

steht,
in welchen

$R^6$ und $R^7$ gleich oder verschieden sind und Wasserstoff, Phenyl oder geradkettiges oder verzweigtes Alkyl mit bis zu 6 Kohlenstoffatomen bedeuten,

$R^8$ Wasserstoff, geradkettiges oder verzweigtes Alkyl mit bis zu 6 Kohlenstoffatomen, Phenyl, Pyridyl, Furyl, Thienyl oder Imidazolyl bedeutet, die gegebenenfalls bis zu 2-fach gleich oder verschieden durch Nitro, Carboxyl, Fluor, Chlor, Brom, Cyano, durch geradkettiges oder verzweigtes Alkoxycarbonyl mit bis zu 4 Kohlenstoffatomen oder durch geradkettiges oder verzweigtes Alkyl mit bis zu 5 Kohlenstoffatomen substituiert sind, das gegebenenfalls durch Hydroxy, Carboxyl oder durch geradkettiges oder verzweigtes Alkoxy oder Alkoxycarbonyl mit jeweils bis zu 5 Kohlenstoffatomen substituiert ist,
und/oder die Cyclen gegebenenfalls durch eine Gruppe der Formel $-OR^{13}$ substituiert sind,
worin

$R^{13}$ für Wasserstoff oder geradkettiges oder verzweigtes Alkyl oder Alkenyl mit jeweils bis zu 4 Kohlenstoffatomen steht,

$R^9$ Carboxyl, Hydroxy, geradkettiges oder verzweigtes Alkoxycarbonyl mit bis zu 4 Kohlenstoffatomen oder eine Gruppe der Formel $-CH_2OH$ oder $-O-CO-R^{14}$ bedeutet,
worin

$R^{14}$ für geradkettiges oder verzweigtes Alkyl mit bis zu 4 Kohlenstoffatomen steht,

$R^{10}$ Wasserstoff bedeutet,
oder

$R^9$ und $R^{10}$ gemeinsam einen Rest der Formel =O oder $=N-R^{15}$ bilden,
worin

$R^{15}$ für Hydroxy, geradkettiges oder verzweigtes Alkoxy mit bis zu 4 Kohlenstoffatomen oder einen Rest der Formel $-NH-CO-NH_2$ oder $-NH-CS-NH_2$ steht,

L eine Gruppe $-SO_2-$ oder -CO- bedeutet,

$R^{11}$ Phenyl, Pyridyl, Thienyl, Furyl oder Pyrimidyl oder geradkettiges oder verzweigtes Alkyl mit bis zu 4 Kohlenstoffatomen bedeutet, das gegebenenfalls durch Pyridyl, Thienyl, Furyl oder Pyrimidyl substituiert ist, wobei alle Ringsysteme gegebenenfalls bis zu 2-fach gleich oder verschieden durch Hydroxy, Phenoxy, Fluor, Chlor, Brom oder durch geradkettiges oder verzweigtes Alkyl oder Alkoxy mit jeweils bis zu 4 Kohlenstoffatomen substituiert sind,

T ein Schwefel- oder Sauerstoffatom bedeutet,

$R^{12}$ Phenoxy, Phenylthio oder geradkettiges oder verzweigtes Alkoxy mit bis zu 4 Kohlenstoffatomen bedeutet

und deren Salze.

Besonders bevorzugt sind Verbindungen der allgemeinen Formel (I),
in welcher

$R^1$ und $R^2$ unter Einbezug der sie verbindenden Doppelbindung gemeinsam einen Phenyl- oder Pyridylring bilden,

$R^3$ und $R^4$ unter Einbezug der sie verbindenden Doppelbindung gemeinsam einen Phenylring oder einen Cyclopenten-, Cyclohexen-, Cyclohepten-, Cycloocten-Rest bilden,
wobei alle unter $R^1/R^2$ und $R^3/R^4$ aufgeführten Ringsysteme gegebenenfalls bis zu 2-fach gleich oder verschieden durch Fluor, Chlor, Brom, Trifluormethyl, Carboxy, Hydroxy, durch geradkettiges oder verzweigtes Alkoxy oder Alkoxycarbonyl mit jeweils bis zu 3 Kohlenstoffatomen oder durch geradkettiges oder verzweigtes Alkyl mit bis zu 3 Kohlenstoffatomen substituiert sind, das seinerseits durch Hydroxy, Methoxy oder Ethoxy substituiert sein kann,

$R^5$ für Wasserstoff, Fluor, Chlor, Brom, Trifluormethyl, Trifluormethoxy oder Methyl steht,

D für Wasserstoff, Cyclopentyl, Cyclohexyl, Cycloheptyl, Cylcooctyl oder für geradkettiges oder verzweigtes Alkyl mit bis zu 6 Kohlenstoffatomen steht,

E für Wasserstoff oder für einen Rest der Formel
$-CO-NR^6R^7$,

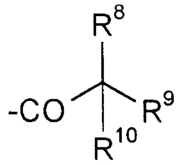 , $L-R^{11}$ oder 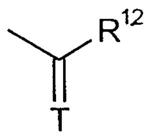

steht,
in welchen

$R^6$ und $R^7$ gleich oder verschieden sind und Wasserstoff, Phenyl oder geradkettiges oder verzweigtes Alkyl mit bis zu 6 Kohlenstoffatomen bedeuten,

$R^8$ Wasserstoff, geradkettiges oder verzweigtes Alkyl mit bis zu 6 Kohlenstoffatomen, Phenyl, Pyridyl oder Thienyl bedeutet, die gegebenenfalls bis zu 2-fach gleich oder verschieden durch Nitro, Carboxyl, Fluor, Chlor, Brom, Cyano, durch geradkettiges oder verzweigtes Alkoxycarbonyl mit bis zu 3 Kohlenstoffatomen oder durch geradkettiges oder verzweigtes Alkyl mit bis zu 4 Kohlenstoffatomen substituiert sind, das gegebenenfalls durch Hydroxy, Carboxyl oder durch geradkettiges oder verzweigtes Alkoxy oder Alkoxycarbonyl mit jeweils bis zu 4 Kohlenstoffatomen substituiert ist,
und/oder die Cyclen gegebenenfalls durch eine Gruppe der Formel $-OR^{13}$ substituiert sind,
worin

$R^{13}$ für Wasserstoff oder geradkettiges oder verzweigtes Alkyl oder Alkenyl mit jeweils bis zu 3 Kohlenstoffatomen steht,

$R^9$ Carboxyl, Hydroxy, geradkettiges oder verzweigtes Alkoxycarbonyl mit bis zu 3 Kohlenstoffatomen oder eine Gruppe der Formel $-CH_2OH$ oder $-O-CO-R^{14}$ bedeutet,
in welcher

$R^{14}$ für geradkettiges oder verzweigtes Alkyl mit bis zu 3 Kohlenstoffatomen steht,

$R^{10}$ Wasserstoff bedeutet,
oder

$R^9$ und $R^{10}$ gemeinsam einen Rest der Formel =O oder $=N-R^{15}$ bilden,
worin

$R^{15}$ Hydroxy, geradkettiges oder verzweigtes Alkoxy mit bis zu 3 Kohlenstoffatomen oder einen Rest der Formel $-NH-CO-NH_2$ oder $-NH-CS-NH_2$ bedeutet,

L einen Rest der Formel $-SO_2$ oder -CO bedeutet,

$R^{11}$ Phenyl, Pyridyl oder Thienyl oder geradkettiges oder verzweigtes Alkyl mit bis zu 3 Kohlenstoffatomen bedeutet, das gegebenenfalls durch Phenyl, Pyridyl oder Thienyl substituiert ist, wobei alle Ringsysteme gegebenenfalls bis zu 2-fach gleich oder verschieden durch Hydroxy, Phenoxy, Fluor, Chlor, Brom oder durch geradkettiges oder verzweigtes Alkyl oder Alkoxy mit jeweils bis zu 3 Kohlenstoffatomen substituiert sind,

T ein Schwefel- oder Sauerstoffatom bedeutet,

$R^{12}$ Phenoxy, Phenylthio oder geradkettiges oder verzweigtes Alkoxy mit bis zu 3 Kohlenstoffatomen bedeutet,

und deren Salze.

Ganz besonders bevorzugt bilden $R^1$ und $R^2$ unter Einbeziehen der Doppelbindung einen Pyridylring. Insbesondere bevorzugt bilden $R^3$ und $R^4$ unter Einbezug der Doppelbindung einen Phenylring.

Ebenso bevorzugt bilden $R^3$ und $R^4$ unter Einbezug oder Doppelbindung einen Cyclopenten oder -hexenring.

Außerdem wurde ein Verfahren zur Herstellung der erfindungsgemäßen Verbindungen der allgemeinen Formel (I), gefunden, dadurch gekennzeichnet, daß man
Carbonsäuren der allgemeinen Formel (II)

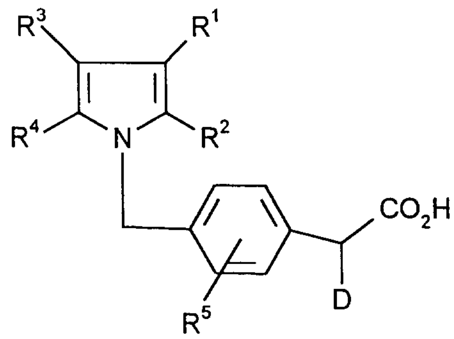

(II)

in welcher
$R^1$, $R^2$, $R^3$, $R^4$, $R^5$ und D die angegebene Bedeutung haben,
zunächst durch Umsetzung mit Phosphorsäurediphenylesterazid in inerten Lösemitteln und in Anwesenheit einer Base in die Amine der allgemeinen Formel (Ia)

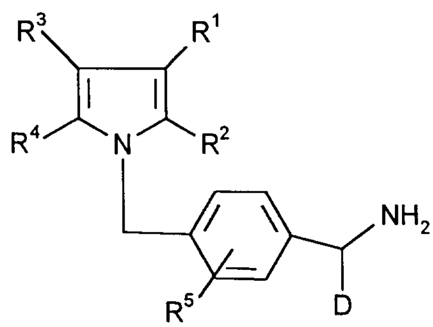

(Ia)

in welcher
$R^1$, $R^2$, $R^3$, $R^4$, $R^5$ und D die angegebene Bedeutung haben,
überführt,
und anschließend mit Verbindungen der allgemeinen Formel (III)

$$E^1\text{-}X \qquad (III)$$

in welcher

$E^1$ die oben angegebene Bedeutung von E hat aber nicht für Wasserstoff steht, und

X in Abhängigkeit von den unter E angegebenen Resten für Hydroxy oder Halogen steht,

in inerten organischen Lösemitteln, gegebenenfalls in Anwesenheit einer Base und/oder von Hilfsmitteln umsetzt, und gegebenenfalls derivatisiert oder variiert.

Die erfindungsgemäßen Verfahren können durch folgendes Formelschema beispielhaft erläutert werden:

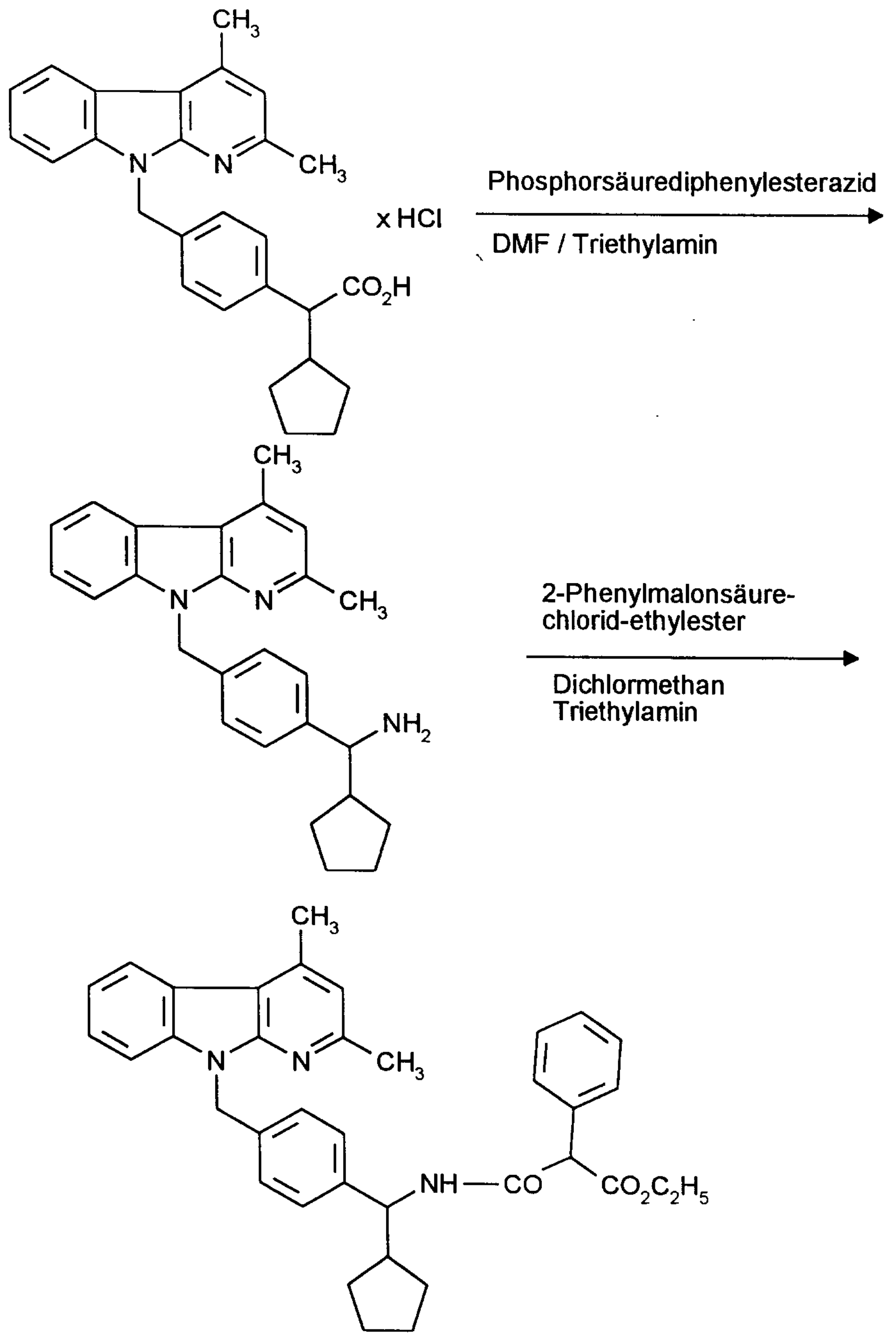


Als Lösemittel für die einzelnen Schritte eignen sich hierbei inerte organische Lösemittel, die sich unter den Reaktionsbedingungen nicht verändern. Hierzu gehören Ether, wie Diethylether oder Tetrahydrofuran, Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohlenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid.

Ebenso ist es möglich, Gemische der Lösemittel einzusetzen. Besonders bevorzugt sind Dichlormethan, Tetrahydrofuran, Aceton oder Dimethylformamid.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid,. Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl($C_1$-$C_6$)amine) wie Triethyl-

amin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle wie Natrium und deren Hydride wie Natriumhydrid einzusetzen. Bevorzugt sind Natrium- und Kaliumcarbonat und Triethylamin.

Die Base wird in einer Menge von 1 mol bis 5 mol, bevorzugt von 1 mol bis 3 mol, bezogen auf 1 mol der Verbindung der allgemeinen Formel (II) eingesetzt.

Die Reaktion wird im allgemeinen in einem Temperaturbereich von 0°C bis 150°C, bevorzugt von +20°C bis +110°C durchgeführt.

Die Umsetzung kann bei normalen, erhöhtem oder bei erniedrigtem Druck durchgeführt werden (z.B. 0,5 bis 5 bar). Im allgemeinen arbeitet man bei Normaldruck.

Die Amidierung kann gegebenenfalls auch über die aktivierte Stufe der Säurehalogenide, die aus den entsprechenden Säuren durch Umsetzung mit Thionylchlorid, Phosphortrichlorid, Phosphorpentachlorid, Phosphortribromid oder Oxalylchlorid hergestellt werden können, verlaufen.

Die oben aufgeführten Basen können gegebenenfalls auch als säurebindende Hilfsmittel für die Amidierung eingesetzt werden.

Als Hilfsmittel eignen sich ebenso Dehydratisieningsreagenzien. Dazu gehören beispielsweise Carbodiimide wie Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Iso-butylchloroformat oder Benzotriazolyloxy-tris-(dimethylamino)phosphoniumhexyl-fluorophosphat oder Phosphonsäurediphenyl-esteramid oder Methan-sulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die Variation von funktionellen Gruppen wie beispielsweise Hydrolyse, Veresterung und Reduktion, sowie die Isomerentrennung und Salzbildung erfolgt nach üblichen Methoden.

Die Verbindungen der allgemeinen Formel (III) sind an sich bekannt oder nach üblichen Methoden herstellbar.

Die Verbindungen der allgemeinen Formel (Ia) sind neu und können wie oben beschrieben hergestellt werden.

Die Carbonsäuren der allgemeinen Formel (II) sind neu und können hergestellt werden, indem man Verbindungen der allgemeinen Formel (IV)

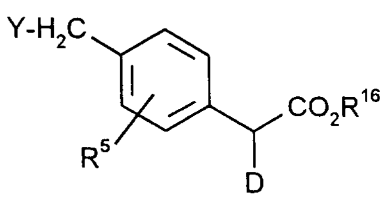

(IV)

in welcher

D und $R^5$ die angegebene Bedeutung haben,

Y für eine typische Abgangsgruppe wie beispielsweise Chlor, Brom, Jod, Tosylat oder Mesylat, vorzugsweise für Brom steht, und

$R^{16}$ für geradkettiges oder verzweigtes Alkyl mit bis zu 4 Kohlenstoffatomen steht,

mit Verbindungen der allgemeinen Formel (V)

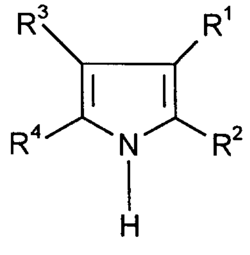

(V)

in welcher
$R^1$, $R^2$, $R^3$ und $R^4$ die angegebene Bedeutung haben
in inerten Lösemitteln, gegebenenfalls in Anwesenheit einer Base, umsetzt.

Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Dimethylformamid und Tetrahydrofuran.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl($C_1$-$C_6$)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle wie Natrium oder deren Hydride wie Natriumhydrid einzusetzen. Bevorzugt sind Natriumhydrid, Kaliumcarbonat, Triethylamin, Pyridin und Kalium-tert.butylat, DBU oder DABCO.

Im allgemeinen setzt man die Base in einer Menge von 0,05 mol bis 10 mol, bevorzugt von 1 mol bis 2 mol, bezogen auf 1 mol der Verbindung der Formel (IV), ein.

Das erfindungsgemäße Verfahren wird im allgemeinen in einem Temperaturbereich von -30°C bis +100°C, bevorzugt von -10°C bis +60°C, durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Verbindungen der allgemeinen Formel (IV) sind bekannt oder können in Analogie zu bekannten Methoden hergestellt werden.

Die Verbindungen der allgemeinen Formeln (V) sind bekannt oder können in Analogie zu bekannten Methoden hergestellt werden.

Die erfindungsgemäßen Verbindungen der allgemeinen Formel (I) und (Ia) haben ein nicht vorhersehbares pharmakologisches Wirkspektrum.

Sie können als Wirkstoffe in Arzneimitteln zur Reduzierung von Veränderungen an Gefaßwänden Verwendung finden und zur Behandlung von koronaren Herzerkrankungen, Herzinsuffizienz, Störungen der Hirnleistung, ischämischen Gehirnerkrankungen, Apoplex, Durchblutungsstörungen, Mikrozirkulationsstörungen und Thrombosen.

Weiterhin spielt bei der Okklusion von Gefäßen die Proliferation glatter Muskelzellen eine ausschlaggebende Rolle. Die erfindungsgemäßen Verbindungen sind geeignet, diese Proliferation zu inhibieren und damit atherosklerotische Prozesse zu verhindern.

Die erfindungsgemäßen Verbindungen zeichnen sich durch eine Senkung der ApoB-100-assoziierten Lipoproteinen (VLDL und seiner Abbauprodukte, wie z.B. LDL), des ApoB-100, der Triglyceride und des Cholesterins aus. Damit besitzen sie wertvolle, im Vergleich zum Stand der Technik überlegene pharmakologische Eigenschaften.

Überraschenderweise besteht die Wirkung der erfindungsgemäßen Verbindungen zunächst in einer Verminderung oder vollständigen Inhibierung der Bildung und/oder der Freisetzung von ApoB-100-assoziierten Lipoproteinen aus Leberzellen, was eine Senkung des VLDL-Plasmaspiegels zur Folge hat. Diese VLDL-Senkung muß mit einer Senkung der Plasmaspiegel von ApoB-100, LDL, Triglyceriden und von Cholesterin einhergehen; es werden also gleichzeitig mehrere der obengenannten Risikofaktoren gesenkt, die an Gefäßwandveränderungen beteiligt sind.

Die erfindungsgemäßen Verbindungen können daher zur Prävention und Behandlung von Atherosklerose, der Fettsucht, Pankreatitis und der Obstipation eingesetzt werden.

**1. Hemmung der Freisetzung ApoB-100-assoziierter Lipoproteine**

Der Test zum Nachweis der Hemmung der Freisetzung ApoB-100-assoziierter Liproproteine aus Leberzellen erfolgte in vitro mit kultivierten Leberzellen, bevorzugt mit Zellen der humanen Linie HepG2. Diese Zellen werden unter Standardbedingungen in Medium für die Kultur eukariontischer Zellen gezüchtet, bevorzugt in RPMI 1640 mit 10% fötalem Kälberserum. HepG2-Zellen synthetisieren und sezernieren in den Kulturüberstand ApoB-100-assoziierte Lipoproteinpartikel, die im Prinzip ähnlich aufgebaut sind wie die VLDL- bzw. LDL-Partikel, die im Plasma zu finden sind.

Diese Partikel können mit einem Immunoassay für humanes LDL nachgewiesen werden. Dieser Immunoassay erfolgt mit Antikörpern, die im Kaninchen gegen humanes LDL unter Standardbedingungen induziert worden waren. Die anti-LDL-Antikörper (Kan-anti-LDL-Ak) wurden an einem Immunosorbens mit humanem LDL affinitätschromatographisch gereinigt. Diese gereinigten Kan-anti-LDL-Ak werden an die Oberfläche von Plastik adsorbiert. Zweckmäßigerweise erfolgt diese Adsorbtion an die Plastikoberfläche von Mikrotitierplatten mit 96 Vertiefungen, bevorzugt an MaxiSorp-Platten. Wenn im Überstand von Hep-G2-Zellen ApoB-100-assoziierte Partikel vorhanden sind, dann können diese an die insolubilisierten Kan-anti-LDL-Ak binden, und es entsteht ein Immunkomplex, der an die Plastikoberfläche gebunden ist. Nicht gebundene Proteine werden durch Waschen entfernt. Der sich an der Plastikoberfläche befindliche Immunkomplex wird mit monoklonalen Antikörpern nachgewiesen, die nach Standardbedingungen gegen humanes LDL induziert und gereinigt worden waren. Diese Antikörper wurden mit dem Enzym Peroxidase konjugiert. Peroxidase setzt das farblose Substrat TMB in Gegenwart von $H_2O_2$ in ein gefärbtes Produkt um. Nach Ansäuerung des Reaktionsgemisches mit $H_2SO_4$ wird die spezifische Lichtadsorption bei 450 nm bestimmt, die ein Maß für die Menge von ApoB-100-assoziierten Partikeln ist, die von den HepG2-Zellen in den Kulturüberstand sezerniert worden waren.

Überraschenderweise hemmen die erfindungsgemäßen Verbindungen die Freisetzung der ApoB-100-assoziierten Partikel. Der $IC_{50}$-Wert gibt an, bei welcher Substanzkonzentration die Lichtadsorption im Vergleich zur Kontrolle (Lösemittelkontrolle ohne Substanz) um 50% inhibiert ist.

| Bsp.-Nr. | $IC_{50}$ [$10^{-9}$ mol/l] |
|---|---|
| 1 | > 2000 |
| 3 | 460 |
| 4 | 34 |
| 5 | 38 |
| 11 | 250 |
| 18 | 39 |
| 24 | 300 |

**2. Bestimmung der VLDL-Sekretion in vivo am Hamster**

Der Effekt der Testsubstanzen auf die VLDL-Sekretion in vivo wird am Hamster untersucht. Hierzu werden Goldhamster nach Prämedikation mit Atropin (83 mg/kg s.c.) mit Ketavet (83 mg/kg s.c.) und Nembutal (50 mg/kg i.p.) narkotisiert. Wenn die Tiere reflexfrei geworden sind, wird die V. jugularis freipräpariert und kanüliert. Anschließend werden 0,25 ml/kg einer 20 %igen Lösung von Triton WR-1339 in physiologischer Kochsalzlösung appliziert. Dieses Detergens hemmt die Lipoproteinlipase und führt so zu einem Anstieg des Triglyceridspiegels aufgrund eines ausbleibenden Katabolismus von sezernierten VLDL-Partikeln. Dieser Triglyceridanstieg kann als Maß für die VLDL-Sekretionsrate herangezogen werden. Den Tieren wird vor sowie ein und zwei Stunden nach Applikation des Detergens durch Punktion des retroorbitalen Venenplexus Blut entnommen. Das Blut wird zwei Stunden bei Raumtemperatur, anschließend über Nacht bei 4°C inkubiert, um die Gerinnung vollständig abzuschließen. Danach wird 5 Minuten bei 10.000 g zentrifugiert. Im so erhaltenen Serum wird die Triglyceridkonzentration mit Hilfe eines modifizierten kommerziell erhältlichen Enzymtests bestimmt (Merckotest® Triglyceride Nr. 14354). 100 µl Serum werden mit 100 µl Testreagenz in 96-Lochplatten versetzt und 10 Minuten bei Raumtemperatur inkubiert. Anschließend wird die optische Dichte bei einer Wellenlänge von 492 nM in einem automatischen Platten-Lesegerät bestimmt (SLT-Spectra). Serumproben mit einer zu hohen Triglyceridkonzentration werden mit physiologischer Kochsalzlösung verdünnt. Die in den Proben enthaltene Triglyceridkonzentration wird mit Hilfe einer parallel gemessenen Standardkurve bestimmt. Testsubstanzen werden in diesem Modell entweder unmittelbar vor Applikation des Detergens intravenös verabreicht oder vor Einleitung der Narkose oral oder subkutan.

**3. Hemmung der intestinalen Triglyceridabsoprtion in vivo (Ratten)**

Die Substanzen, die auf ihre triglyceridabsorptionshemmende Wirkung in vivo untersucht werden sollen, werden männlichen Wistar-Ratten mit einem Körpergewicht zwischen 170 und 230 g oral verabreicht. Zu diesem Zweck werden die Tiere 18 Stunden vor der Substanzapplikation in Gruppen zu 6 Tieren eingeteilt und anschließend wird ihnen das Futter entzogen. Trinkwasser steht den Tieren ad libitum zur Verfügung. Die Tiere der Kontrollgruppen erhalten eine wäßrige Traganth-Suspension bzw. eine Traganth-Suspension die Olivenöl enthält. Die Traganth-Olivenöl-Suspension wird mit dem Ultra-Turrax hergestellt. Die zu untersuchenden Substanzen werden in einer entsprechenden Traganth-Olivenöl-Suspension ebenfalls mit dem Ultra-Turrax, direkt vor der Substanzapplikation suspendiert.

Jeder Ratte wird vor der Schlundsondenapplikation zur Bestimmung des basalen Serumtriglyceridgehaltes Blut durch Punktion des retroorbitalen Venenplexus' entnommen. Anschließend werden die Traganth-Suspension, die Traganth-Olivenöl-Suspensionen ohne Substanz (Kontrolltiere), bzw. die Substanzen, suspendiert in einer entsprechenden Traganth-Olivenöl-Suspension, den nüchternen Tieren mit einer Schlundsonde verabreicht. Die weiteren Blutentnahmen zur Bestimmung des postprandialen Serumtriglyceridanstiegs erfolgen in der Regel 1, 2 und 3 Stunden nach der Schlundsondenapplikation.

Die Blutproben werden zentrifugiert und nach Gerinnung des Serums die Triglyceride photometrisch mit einem EPOS-Analyzer 5060 (Eppendorf Gerätebau, Netheler & Hinz GmbH, Hamburg) bestimmt. Die Bestimmung der Trigylceride erfolgt vollenzymatisch mit einem handelsüblichen UV-Test.

Der postprandiale Serumtriglyceridanstieg wird durch Subtraktion des Triglyceridvorwertes jeden Tieres von seinen korrespondierenden postprandialen Triglyceridkonzentrationen (1, 2 und 3 Stunden nach Applikation) ermittelt.

Die Differenzen (in mmol/l) zu jedem Zeitpunkt (1, 2 und 3 Stunden) werden in den Gruppen gemittelt, und die Mittelwerte des Serumtriglyceridanstiegs (ΔTG) der substanzbehandelten Tiere mit den Tieren verglichen, die nur die Traganth-Öl-Suspension erhielten.

Ebenso wird der Serumtriglyceridverlauf der Kontrolltiere, die nur Traganth erhielten, berechnet. Der Substanzeffekt zu jedem Zeitpunkt (1, 2 oder 3 Stunden) wird wie folgt ermittelt und in Δ% von der ölbelasteten Kontrolle angegeben.

$$\Delta\%\ \text{Triglyceridanstieg} = \frac{\Delta TG_{\text{Substanz}} - \Delta TG_{\text{Traganthkontrolle}}}{\Delta TG_{\text{Ölbelastung}} - \Delta TG_{\text{Traganthkontrolle}}} \times 100$$

Effekt von 10 mg Prüfsubstanz / kg KG p.o. auf den Triglyceridanstieg (Δ%) 2 h nach einer Triglyceridbelastung im Serum nüchterner Ratten. Der Serumtriglyceridanstieg fettbelasteter Kontrolltiere bezogen auf den Serumtriglyceridspiegel von Traganth-Kontrolltieren entspricht 100%. n = 6 Tiere pro Gruppe.

Die statistische Auswertung erfolgt mit Student's t-Test nach vorheriger Überprüfung der Varianzen auf Homogenität.

Substanzen, die zu einem Zeitpunkt den postprandialen Serumtriglyceridanstieg, verglichen mit dem der unbehandelten Kontrollgruppe, statistisch signifikant ($p < 0{,}05$) um mindestens 30 % vermindern, werden als pharmakologisch wirksam angesehen.

**4. Hemmung der VLDL-Sekretion in vivo (Ratte)**

Die Wirkung der Testsubstanzen auf die VLDL-Sekretion wird ebenfalls an der Ratte untersucht. Dazu wird Ratten 500 mg/kg Körpergewicht (2,5 mg/kg) Triton WR-1339, gelöst in physiologischer Kochsalzlösung, intravenös in die Schwanzvene appliziert. Triton WR-1339 inhibiert die Lipoproteinlipase und führt somit durch Hemmung des VLDL-Katabolismus zu einem Anstieg des Triglycerid- und Cholsterinspiegels. Diese Anstiege können als Maß für die VLDL-Sekretionsrate herangezogen werden.

Den Tieren wird vor sowie eine und zwei Stunden nach Applikation des Detergens durch Punktion des retroorbitalen Venenplexus Blut entnommen. Das Blut wird zur Gerinnung 1 h bei Raumtemperatur inkubiert und das Serum durch Zentrifugation mit 10 000 g für 20 s gewonnen. Anschließend werden die Triglyceride mittels eines handelsüblichen gekoppelten Enzymtests (Sigma Diagnostics®, Nr. 339) bei einer Wellenlänge von 540 nm photometrisch bestimmt. Die Messung erfolgt mit Hilfe eines ebenfalls gekoppelten Enzymtests (Boehringer Mannheim®, Nr. 1442350) bei einer Wellenlänge von 546 nm. Proben mit Triglycerid- bzw. Cholesterinkonzentrationen, die den Meßbereich der Methoden überschreiten, werden mit physiologischer Kochsalzlösung verdünnt. Die Ermittlung der jeweiligen Serumkonzentrationen erfolgt anhand parallel gemessener Standardreihen. Testsubstanzen werden unmittelbar nach der Tritoninjektion oral, intravenös oder subcutan appliziert.

Die Erfindung betrifft außerdem die Kombination von Amiden und Sulfonamiden heterocyclisch substituierter Benzylamine der allgemeinen Formel (I) mit einem Glucosidase- und/oder Arylasehemmer zur Behandlung von familiärer Hyperlipidämien, der Fettsucht (Adipositas) und des Diabetes mellitus. Glucosidase- und/oder Arylasehemmer im Rahmen der Erfindung sind beispielsweise Acarbose, Adiposine, Voglibose (AO-128), Miglitol, Emiglitate, MDL-25637, Camiglibase (MDL-73945), Tendamistete, AI-3688, Treslatine, Pradimicin-Q und Salboslatine. Bevorzugt ist die Kombination von Acarbose, Miglitol, Emiglitate oder Voglibose mit einer der oben aufgeführten erfindungsgemäßen Verbindungen der allgemeinen Formel (I).

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

**Verwendete Abkürzungen:**

| | |
|---|---|
| Ac = | Acetyl |
| Bn = | Benzyl |
| Bz = | Benzoyl |
| cDec = | cyclo Decyl |
| cDodec = | cyclo Docecyl |
| cHept = | cyclo Heptyl |
| cHex = | cyclo Hexyl |
| cNon = | cyclo Nonyl |
| cOct = | cyclo Octyl |
| cPent = | cyclo Pentyl |
| cPr = | cyclo Propyl |
| cUndec = | cyclo Undecyl |
| DCC = | Dicyclohexylcarbodiimid |
| DDQ = | 2,3-Dichlor-5,6-dicyano-1,4-benzochinon |
| dia = | Diastereomer |
| DMAP = | 4-(N,N-Dimethylamino)pyridin |
| DMF = | N,N-Dimethylformamid |
| DMSO = | Dimethylsulfoxid |
| ent = | Enantiomer |
| Et = | Ethyl |
| HOBT = | 1-Hydroxy-1H-benzotriazol |
| iBu = | iso Butyl |
| iPr = | iso Propyl |
| Me = | Methyl |
| Mes = | Mesyl |
| NBS = | N-Bromsuccinimid |
| nBu = | normal Butyl |
| nPr = | normal Propyl |
| Ph = | Phenyl |
| PPA = | Polyphosphorsäure |
| pTol = | para Tolyl |
| pTos = | para Tosyl |
| rac = | Racemat |
| sBu = | sekundär Butyl |
| tBu = | tertiär Butyl |
| TFA = | Trifluoressigsäure |
| THF = | Tetrahydrofuran |
| TMS = | Tetramethylsilan |

| Solvens | Bezeichnung |
|---|---|
| Dichlormethan : Ethanol = 20:1 | A |
| Dichlormethan : Ethanol = 50:1 | B |
| Dichlormethan : Ethanol = 100:1 | C |
| Petrolether : Essigsäureethylester = 1:1 | D |
| Dichlormethan : Methanol = 50:1 | E |
| Petrolether : Essigsäureethylester = 5:1 | F |

**Ausgangsverbindungen**

**Beispiel I**

6-Chlor-2,4-lutidin (2-Chlor-4,6-dimethyl-pyridin)

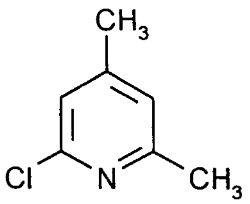

Zur Darstellung der Titelverbindung [US 36 32 807] werden 600 g (4,91 mol) 6-Amino-2,4-lutidin in 2 l Methanol gelöst und die Lösung bei ca. 0°C mit Chlorwasserstoffgas gesättigt. Bei einer Innentemperatur unter 10°C werden 1,307 l (9,82 mol) Isopentylnitrit zugetropft (ca. 2,5 h) und das Gemisch unter Erwärmung auf Raumtemperatur (ca. 25°C) 15 h so belassen. Die Lösung wird im Vakuum weitgehend vom Lösemittel befreit, mit 3 l Dichlormethan und 1,5 l Wasser versetzt und unter Kühlung (< 20°C) mit konzentrierter wäßriger Ammoniaklösung auf einen pH = 9,5 gestellt. Die abgetrennte organische Phase wird mit Natriumsulfat getrocknet, zuerst am Rotationsverdampfer im Vakuum eingeengt und dann über eine Vigreux-Kolonne destilliert:
Fraktion 1) Kp. = 47-49°C (12 Torr), 603 g
Fraktion 2) Kp. = 82-85°C (12 Torr), 612 g (ca. 88% roh)
$R_f$ = 0,39 (Petrolether : Essigester = 10:1)
Das rohe Produkt, das kleine Mengen 6-Methoxy-2,4-lutidin enthalten kann, wird ohne weitere Reinigung weiter umgesetzt.

**Beispiel II**

6-Hydrazino-2,4-lutidin (4,6-Dimethyl-2-hydrazino-pyridin)

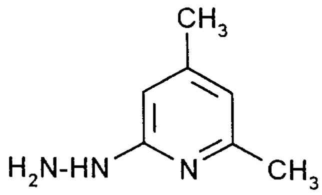

580 g (4,10 mol) der Verbindung aus Beispiel I werden in 800 ml Diethylenglycol gelöst und mit 1050 ml Hydrazin-Hydrat 48 h bei einer Badtemperatur von ca. 140°C gerührt. Der abgekühlte Ansatz wird auf 4,5 l Ether und 4,5 l Wasser gegossen und die organische Phase wird zweimal mit je 2,3 l Dichlormethan extrahiert. Die vereinigten organischen Phasen werden mit Natriumsulfat getrocknet und im Vakuum eingedampft. Es fallen 784 g lösemittelhaltiges Rohprodukt an, das ohne Aufarbeitung weiter umgesetzt wird.
$R_f \approx 0,37$ (Dichlormethan : Methanol = 10:1)

**Beispiel III**

2,4-Dimethyl-5,6,7,8-tetrahydro-α-carbolin

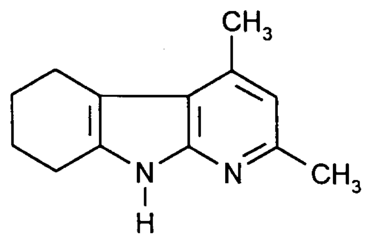

78 g (max. 0,49 mol) der rohen Verbindung aus Beispiel II werden bei Raumtemperatur (ca. 25°C) mit 59 ml (0,56 mol) Cyclohexanon umgesetzt, wobei die Innentemperatur ansteigt. Nach 2 h ist das Edukt verschwunden (DC-Kontrolle; Dichlormethan : Methanol = 10:1). Die Mischung wird in 40 ml Diethylenglycol aufgenommen und unter Rückfluß umgesetzt, dabei werden tiefer als das Lösemittel siedende Bestandteile (z.B. Reaktionswasser und überschüssiges Cyclohexanon) destillativ entfernt (Wasserabscheider). Nach 3 h ist das intermediäre Hydrazon verschwunden (DC-Kontrolle; Petrolether:Essigester = 1:1); das Reaktionsgemisch wird auf Raumtemperatur abgekühlt und mit Aceton verrührt Der anfallende Niederschlag wird abgesaugt, mit Aceton nachgewaschen und im Vakuum getrocknet (34,4 g). Die vom Lösemittel weitgehend befreiten Mutterlaugen werden wiederum mit Aceton behandelt, wobei weitere 9,3 g Produkt anfallen (Gesamtausbeute über 3 Stufen: 43,7 g / 0,22 mol / 47%).
Fp.: 248°C (unkorrigiert)
$R_f = 0,41$ (Dichlormethan : Ethanol = 20:1)

**Beispiel IV**

2,4-Dimethyl-α-carbolin

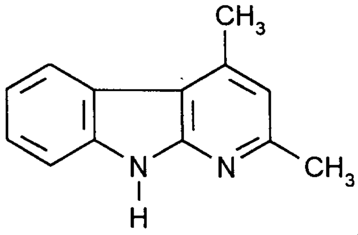

100 g (499 mmol) der Verbindung aus Beispiel III werden in 700 ml Diethylenglykol mit 164 ml (1 mol) Fumarsäurediethylester an 52 g Palladium (5% auf Kohle) unter Rückfluß umgesetzt. Bei der hohen Innentemperatur destilliert eine kleine Menge Ethanol ab (ggf. Wasserabscheider verwenden). Nach ca. 8 h ist das Edukt verschwunden (DC-Kontrolle; Petrolether : Essigester = 1:1, Detektion in der Jodkammer). Das abgekühlte Gemisch wird mit 3 l Aceton versetzt, aufgekocht, heiß über einen Klarfilter (Fa. Seitz) abgesaugt und mit 1 l heißem Aceton nachgewaschen. Beim Abkühlen fällt ein Niederschlag an, der nach Absaugen, Spülen mit kaltem Aceton und Trocknen im Vakuum 58,3 g Produkt liefert. Die Mutterlauge wird im Vakuum weitgehend vom Aceton befreit, wobei der ausfallende Niederschlag wie oben aufgearbeitet wird (9,4 g). Das Filtrat wird wiederum vom Aceton befreit; nach Zugabe von n-Pentan fällt ein weiteres Mal Produkt aus (3,1 g / Aufarbeitung s.o.); Gesamtausbeute: 72%.
Fp.: 220-221°C (unkorrigiert)
$R_f$ = 0,47 (Petrolether : Essigester = 1:1)

**Beispiel V**

4-Methylphenyl-essigsäure-tert.butylester

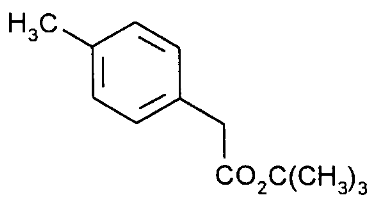

450 g (3 mol) 4-Methylphenyl-essigsäure (Aldrich), 1,13 l (12 mol) tert.Butanol und 90 g (0,74 mol) 4-(N,N-Dimethylamino)pyridin werden in 2 l Dichlormethan gelöst. Nach Zugabe von 680 g (3,3 mol) Dicylcohexylcarbodiimid, gelöst in 400 ml Dichlormethan, wird 20 h bei 25°C gerührt, der ausgefallene Harnstoff abgesaugt, mit 200 ml Dichlormethan gewaschen und die organische Phase je zweimal mit 500 ml 2 M Salzsäure und Wasser gewachen. Die organische Phase wird eingeengt und destilliert.
Ausbeute: 408 g (66% d.Th.)
Kochpunkt: 73-78°C / 0,2 mm

**Beispiel VI**

2-(R,S)-2-Cyclopentyl-2-(4-methylphenyl)essigsäure-tert.butylester

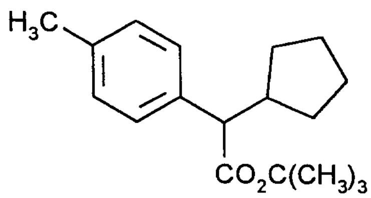

33,5 g (0,3 mol) Kalium-tert.butylat werden in 100 ml wasserfreiem DMF bei 0°C vorgelegt, und 51,6 g (0,25 mol) der Verbindung aus Beispiel V in 250 ml wasserfreiem DMF zugetropft. Es wird 30 min. bei 0°C gerührt und 32,2 ml (0,3 mol) Cyclopentylbromid in 150 ml wasserfreiem DMF bei 5-15°C zugetropft und 20 h bei 25°C gerührt. Nach Einengen wird der Rückstand zwischen Wasser und Diethylether verteilt, die Etherphase über Natriumsulfat getrocknet und eingeengt. Das Produkt kristallisiert aus.
Ausbeute: 67 g (97,5% d.Th.)
Festpunkt = 51-53°C

**Beispiel VII**

2-(R,S)-2-(4-Brommethyl-phenyl)-2-cyclopentyl-essigsäure-tert.butylester

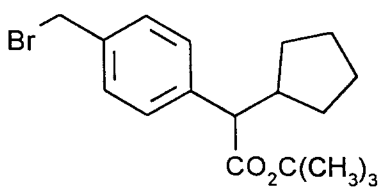

27,4 g (0,1 mol) der Verbindung aus Beispiel VI werden in 200 ml Tetrachlormethan gelöst und zum Sieden erhitzt. Nach Zugabe von 0,82 g Azobisisobutyronitril werden 18,7 g (0,105 mol) N-Bromsuccinimid portionsweise zugegeben und anschließend 1 h refluxiert, auf 0°C abgekühlt und vom Succinimid abfiltiert. Nach Einengen des Filtrates fällt das Produkt aus. Es wird mit Petrolether (40/60) gewaschen und getrocknet.
Ausbeute: 20 g (57% d.Th.)
Fp. = 73-76°C

**Beispiel VIII**

2(R,S)-2-Cyclopentyl-2-[4-(2,4-dimethyl-α-carbolin-9-yl)methyl]phenyl-essigsäure-tert.butylester

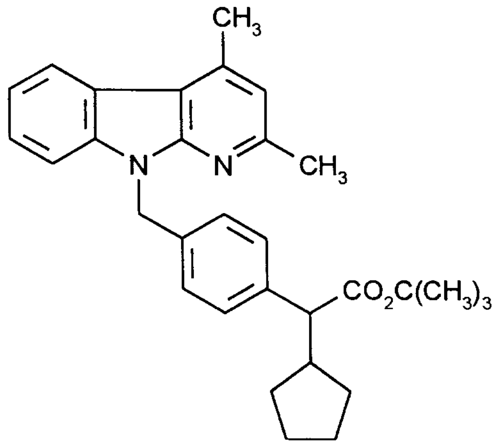

73,6 g (375 mmol) der Verbindung aus Beispiel IV werden bei 25°C in 700 ml wasserfreiem N,N-Dimethylformamid mit 42,13 g (375 mmol) Kalium-tert.butanolat 30 min. umgesetzt und dann mit 161,7 g (375 mmol) der Verbindung aus Beispiel VII, gelöst in 680 ml wasserfreiem N,N-Dimethylformamid, versetzt. Nach 1 h ist die Umsetzung beendet (DC-Kontrolle; Petrolether : Essigester = 10:1). Zur Aufarbeitung werden 2 l Pufferlösung (pH = 4 / Fa. Merck) und 2 l Wasser zugegeben, der anfallende Niederschlag abgesaugt, mit Wasser gewaschen und wieder scharf abgesaugt. Der mäßig feuchte Feststoff wird nun nacheinander mit Petrolether und Methanol verrührt und abgesaugt. Die Vakuumtrocknung über Phosphorpentoxid liefert 139,8 g (298 mmol / 79%) Produkt.
Fp. = 160-161°C (unkorrigiert)
$R_f$ = 0,39 (Petrolether : Essigester = 10:1)

**Beispiel IX**

2-(R,S)-2-Cyclopentyl-2-[4-(2,4-dimethyl-α-carbolin-9-yl)methyl]phenylessigsäure Hydrochlorid

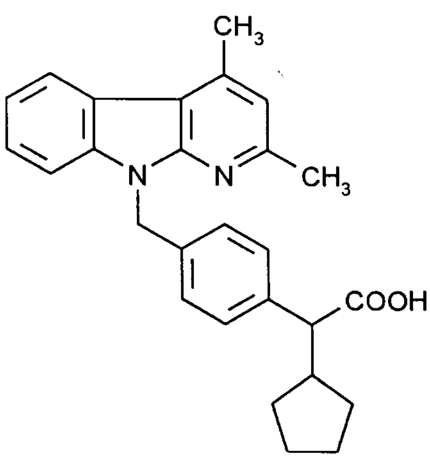

· x-HCl

139,8 g (298 mmol) der Verbindung aus Beispiel VIII werden in 1 l 1,4-Dioxan gelöst und 3 h mit 240 ml konzentrierter Salzsäure (37%ig) bei 70°C gerührt. Nach beendeter Umsetzung (DC-Kontrolle; Petrolether : Essigester = 10:1) wird der Ansatz auf ca. 15°C gekühlt und dann portionsweise auf 5 l Wasser gegossen. Der pH-Wert wird mit 2 M wäßriger Natronlauge auf 2,8 gestellt, der anfallende Niederschlag über einen Papierfilter abgesaugt und mit Wasser so oft nachgewaschen, bis das Waschwasser einen pH > 4 besitzt. Der scharf abgesaugte Feststoff wird mit 1 l Petrolether (Siedebereich 60-80°C) verrührt, wiederum abgesaugt und im Vakuum über Phosphorpentoxid getrocknet.
Ausbeute: 130,3 g (290 mmol / 97%)
Fp. = 260-262°C (unkorrigiert)
$R_f$ = 0,51 (Dichlormethan : Ethanol = 20:1)

## Herstellungsbeispiele

### Beispiel 1

9-{4-[1-(R,S)-1-Amino-1-cyclopentyl-methyl]phenyl-methyl}-2,4-dimethyl-α-carbolin

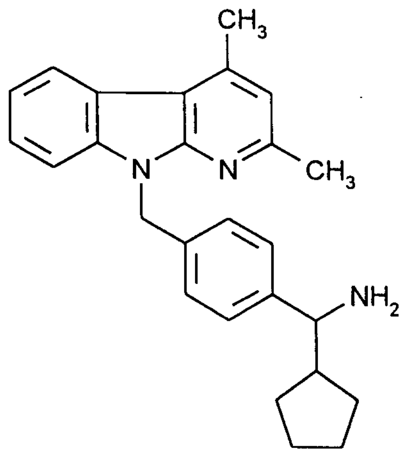

20,0 g (48,5 mmol) der Verbindung aus Beispiel IX werden in 160 ml wasserfreiem DMF gelöst und bei 0°C mit 12,4 ml (58,2 mmol) Phosphorsäurediphenylesterazid und 16,1 ml (116,4 mmol) Triethylamin versetzt. Die Reaktionsmischung wird eine Stunde bei 70°C gerührt, auf 0°C abgekühlt, langsam mit 62,5 ml 6 M Salzsäure versetzt und weitere 2,5 Stunden bei 70°C gerührt. Der Ansatz wird auf 2 l Wasser gegossen und auf einen pH-Wert von 2 gestellt. Der angefallene Niederschlag wird abgesaugt, nacheinander mit Wasser und Petrolether gewaschen und jeweils scharf abgesaugt. Nach der Trocknung über Phosphorpentoxid im Hochvakuum liegt ein Rohprodukt vor, das mit Dichlormethan verrührt, abgesaugt und im Hochvakuum getrocknet 13,6 g (35,5 mmol) des sauberen Produktes liefert.

$R_f$ = 0,35 (Dichlormethan : Ethanol = 5:1)

MS (FAB): m/z = 384 (49% $[M+H]^+$), 367 (100%, $M^+$-$NH_2$), 314 (31%, $M^+$-cPent).

### Beispiel 2

9-{4-[1-(R,S)-1-(2-(R,S)-2-Phenyl-2-ethoxycarbonyl-acetyl)amino-1-cyclopentylmethyl]phenyl-methyl}-2,4-dimethyl-α-carbolin

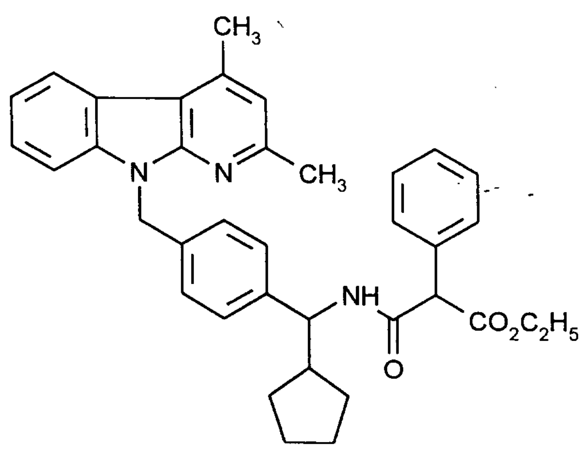

1,0 g (2,6 mmol) der Verbindung aus Beispiel 1 werden in 60 ml Dichlormethan mit 1,1 ml (7,8 mmol) Triethylamin versetzt und bei 20°C mit einer Lösung von 0.89 g (2,7 mmol) 2-(R,S)-2-Phenyl-malonsäure-chlorid-ethylester in 5 ml Dichlormethan zur Reaktion gebracht. Nach 3 Stunden setzt man Puffer (Merck) vom pH = 4 zu, trennt die Phasen und extrahiert mit Puffer und Wasser nach. Die organische Phase wird mit Magnesiumsulfat getrocknet, eingedampft und der erhaltene Rückstand an Kieselgel 60 (Merck, Dichlormethan : Ethanol = 100:1) chromatographisch gereinigt.
Ausbeute: 0,75 g
$R_f$ = 0,32 (Dichlormethan : Ethanol = 50:1)
MS (FAB): m/z = 596 (11%, $[M+Na]^+$), 574 (100%, $[M+H]^+$), 504 (9%, $[M-C_5H_9]^+$).

Die Verbindungen der Tabelle 1 werden analog der Vorschrift von Beispiel 2 hergestellt:

**Tabelle 1:**

* racemisch

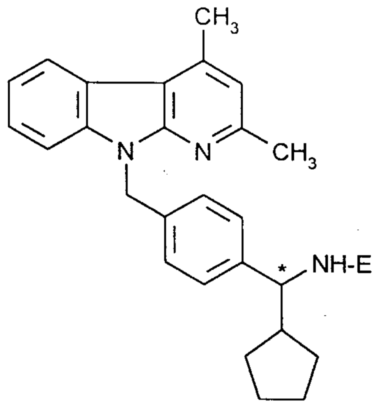

| Bsp.-Nr. | -E | $R_f$ (Solvens) | MS | Ausgangsmaterial (Bsp.-Nr.) |
|---|---|---|---|---|
| 3 | $-SO_2CH_2C_6H_5$ | 0,56 (B) | | 1 |
| 4 | $-CO-CH_2C_6H_5$ | 0,25 (B) | | 1 |
| 5 | 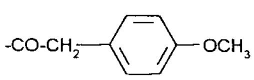 | 0,27 (B) | | 1 |
| 6 | 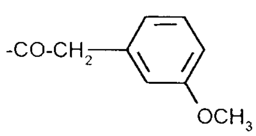 | 0,23 (B) | | 1 |
| 7 | 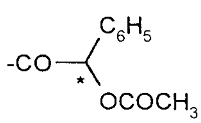 * rac | 0,28 (B) | | 1 |
| 8 | 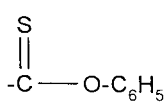 | 0,56 (C) | | 1 |
| 9 | $-SO_2-C_6H_5$ | 0,20 (C) | | 1 |
| 10 | $-CO-C_6H_5$ | 0,14 (C) | | I |
| 11 | 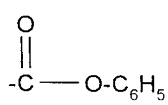 | 0,25 (C) | | 1 |

**Tabelle 1:** (Fortsetzung)

| Bsp.-Nr. | -E | $R_f$ (Solvens) | MS/MP | Ausgangsmaterial (Bsp.-Nr.) |
|---|---|---|---|---|
| 12 | 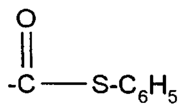 | 0,55 (B) | | 1 |
| 13 | $-CO(CH_2)_2-C_6H_5$ | 0,40 (A) | | 1 |
| 14 | 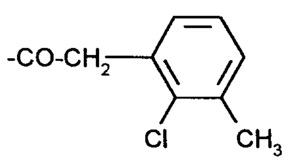 | 0,49 (A) | | 1 |
| 15 | 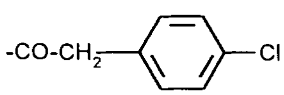 | 0,37 (A) | | 1 |
| 16 | 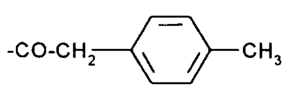 | 0,41 (E) | 516 (100%)/ 222°C | 1 |

**Beispiel 17**

9-{4-[1-(R,S)-1-(2-(R,S)-2-Phenyl-2-hydroxymethyl-acetyl)amino-1-cyclopentyl-methyl]phenyl-methyl}-2,4-dimethyl-α-carbolin

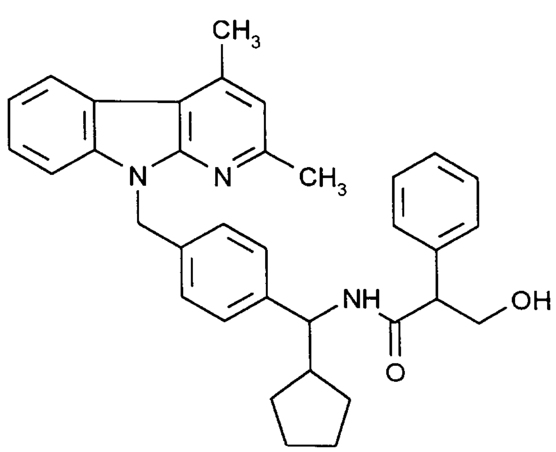

688 mg (1,2 mmol) der Verbindung aus Beispiel 2 werden in 10 ml THF gelöst und bei 0°C mit 2,4 ml (2,4 mmol) 1 M Lithiumalanatlösung in THF zur Reaktion gebracht, wobei man die Innentemperatur auf 20°C ansteigen läßt. Nach 4

Stunden ist die Reaktion noch nicht beendet, man setzt weitere 1,2 ml (1,2 mmol) 1 M Lithiumalanatlösung in THF zu und rührt weitere 3 Stunden bei 20°C. Darauf gießt man das Gemisch auf Puffer von pH = 4 (Merck) und Essigester, trennt die Phasen und extrahiert die wäßrige Phase mehrfach mit Essigester nach. Die vereinigten organischen Extrakte werden mit Magnesiumsulfat getrocknet und eingedampft. Das Rohprodukt wird chromatographisch an Kieselgel 60 (Merck, Dichlormethan: Ethanol = 100:1) gereinigt.
Ausbeute: 240 mg
$R_f$ = 0,11 (Dichlormethan : Ethanol = 50:1)
MS (FAB): m/z = 532 (49%, $[M+H]^+$).

**Beispiel 18**

9-{4-[1-(R,S)-1-(2-(R,S)-2-Phenyl-2-hydroxy-acetyl)amino-1-cyclopentylmethyl]phenyl-methyl}-2,4-dimethyl-α-carbolin

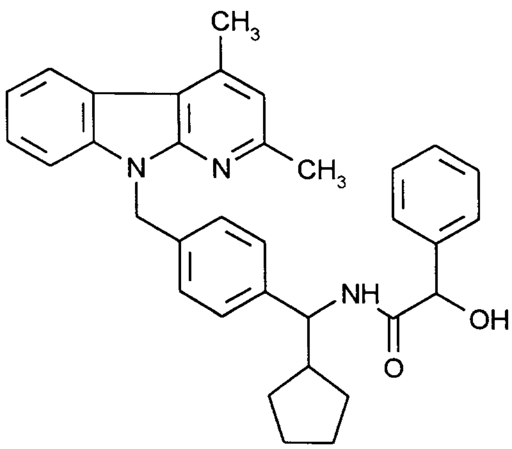

600 mg (1,07 mmol) der Verbindung aus Beispiel 7 werden in 10 ml Ethanol mit 10 ml 2 M wäßriger Natronlauge bei 40°C 2 Stunden umgesetzt. Das Reaktionsgemisch wird mit 40 ml Wasser verdünnt und langsam bei 0°C mit 2 M Salzsäure auf einen pH-Wert von 5,6 gestellt. Der angefallene Niederschlag wird abgesaugt, mit Wasser gewaschen und im Hochvakuum über Phosphorpentoxid getrocknet.
Ausbeute: 485 mg
$R_f$ = 0,63 (Dichlormethan : Ethanol = 20:1)
MS (FAB): m/z = 540 (28%, $[M+Na]^+$), 518 (100%, $[M+H]^+$).

Beispiel 19

9-{4-[1-(R,S)-1(2-Thienyl-acetyl)amino-1-cyclopentyl-methyl]phenyl-methyl}-2,4-dimethyl-α-carbolin

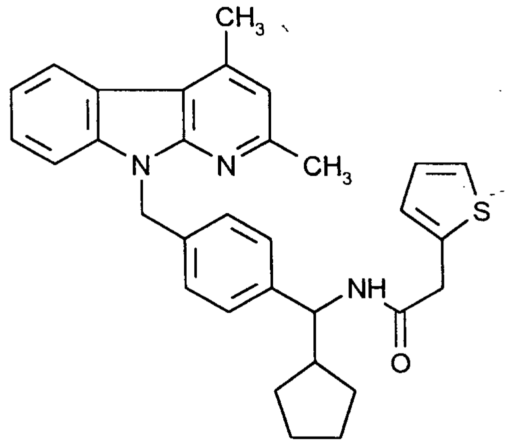

500 mg (1,3 mmol) der Verbindung aus Beispiel 1 werden bei 10°C in 60 ml Dichlormethan mit 193,7 mg (1,43 mmol) HOBT, 287,4 mg (1,5 mmol) N'-(3-Dimethylaminopropyl)-N-ethylcarbodiimid Hydrochlorid, 361 µl (2,61 mmol) Triethylamin und 184,8 mg (1,3 mmol) Thienylessigsäure versetzt und 20 Stunden unter Erwärmung auf 20°C gerührt. Die Reaktionsmischung wird mehrfach mit Salzsäure von pH = 4, darauf mit wäßriger Ammoniumchloridlösung, mit wäßriger Natriumhydrogencarbonatlösung und mit Wasser extrahiert, mit festem wasserfreiem Magnesiumsulfat getrocknet und eingedampft. Das Rohprodukt wird mit Methanol verrührt, abgesaugt, noch einmal mit Methanol nachgewaschen und im Hochvakuum über Phosphorpentoxid getrocknet.

Ausbeute: 400 mg

$R_f$ = 0,35 (Dichlormethan : Ethanol = 20:1)

Die Verbindungen der Tabelle 2 werden analog der Vorschrift von Beispiel 19 hergestellt:

**Tabelle 2:**

* racemisch

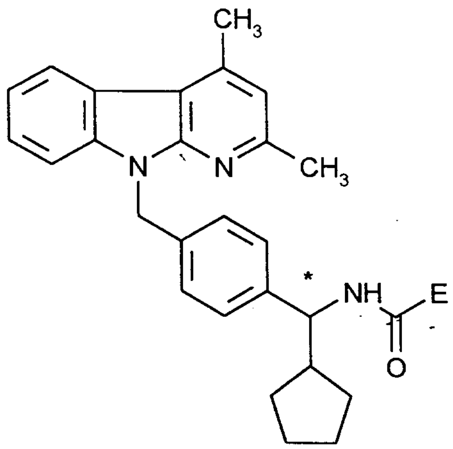

| Bsp.-Nr. | E | $R_f$ (Solvens) | MS / Fp. | Ausgangsmaterial (Bsp.-Nr.) |
|---|---|---|---|---|
| 20 | 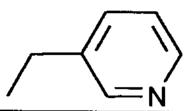 | 0,26 (A) | | 1 |
| 21 | 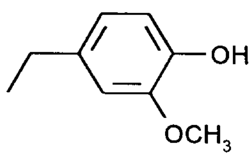 | 0,38 (A) | | 1 |
| 22 | $-CO-C_6H_5$ | 0,26 (F) | 516 (35%) | 1 |

**Beispiel 23**

9-{4-[1-(R,S)-1-Phenylaminocarbonylamino-1-cyclopentyl-methyl]phenyl-methyl}-2,4-dimethyl-α-carbolin

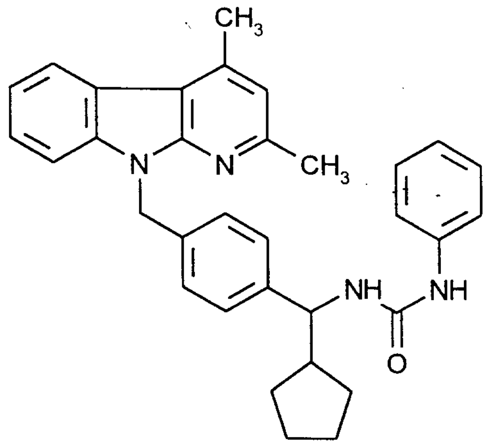

400 mg (1,04 mmol) der Verbindung aus Beispiel 1 werden in 60 ml wasserfreiem Dichlormethan nach Zusatz von 433 µl (3,13 mmol) Triethylamin bei 20°C mit 117,6 mg (0,988 mmol) Phenylisocyanat umgesetzt. Nach 3 Stunden gibt man 120 ml Puffer (Merck) vom pH = 4 dazu, wäscht die organische Phase zweimal mit Wasser, trocknet sie mit Magnesiumsulfat und dampft sie zur Trocke ein. Der Rückstand wird mit Methanol verrührt, abgesaugt und noch einmal mit Methanol gewaschen. Die anschließende Trocknung im Hochvakuum über Phosphorpentoxid liefert 385 mg Produkt.
$R_f$ = 0,54 (Dichlormethan : Ethanol = 20:1)
MS (FAB): m/z = 503 (14%, $[M+H]^+$), 307 (100%).

**Beispiel 24**

9-{4-[1-(R,S)-1-(2-(E,Z)-2-Hydroxyimino-2-phenyl-acetyl)amino-1-cyclopentyl-methyl]phenyl-methyl}-2,4-dimethyl-α-carbolin

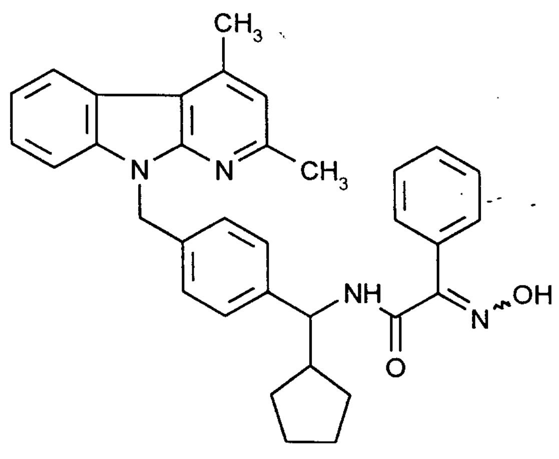

200 mg (0,39 mmol) der Verbindung aus Beispiel 22 werden in 10 ml Pyridin mit 53,9 mg (0.78 mmol) Hydroxylammoniumchlorid versetzt und 20 Stunden unter Rückfluß gekocht. Die abgekühlte Reaktionsmischung wird auf wäßrigen Puffer (pH = 2 / Merck) und Ether gegossen, die Phasen getrennt und die wäßrige Phase mehrfach mit Ether nachextrahiert. Darauf trocknet man mit Magnesiumsulfat und dampft ein. Die chromatographische Aufreinigung (Kieselgel 60 / Merck / Petrolether : Essigsäureethylester = 5 : 1 bis 2 : 1) liefert eine Ausbeute von 135 mg Substanz.
$R_f$ = 0,24 und 0,34 (Petrolether : Essigsäureethylester = 2:1)
MS (FAB): m/z = 531 (100%, $[M+H]^+$), 367 (32%, $M^+$-NHCOC(Ph)=NOH).

Die Verbindungen der Tabelle 3 werden analog der Vorschrift von Beispiel 24 hergestellt:

Tabelle 3:

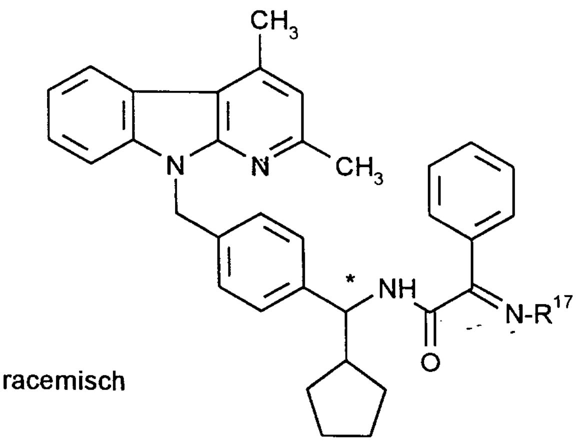

* racemisch

| Bsp.-Nr. | $R^{17}$ | Isomer | $R_f$ (Solvens) | MS-FAB | Ausgangs-material Bsp.-Nr. |
|---|---|---|---|---|---|
| 25 | $-OCH_3$ | E+Z | 0,28 (B) | 545 (50%) | 22 |
| 26 | $-NH-CO-NH_2$ | E+Z | 0,49/ 0,52 (A) | 573 (100%) | 22 |
| 27 | $-NH-CS-NH_2$ | E+Z | 0,48/ 0,53 (A) | 589 (29%) | 22 |

**Patentansprüche**

**1.** Amide und Sulfonamide von heterocyclisch substituierten Benzylaminen der allgemeinen Formel (I)

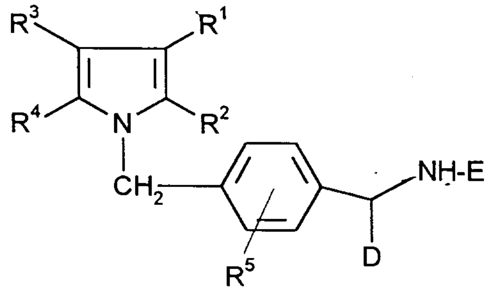

(I)

in welcher

$R^1$ und $R^2$ unter Einbezug der sie verbindenden Doppelbindung gemeinsam einen Phenyl- oder Pyridylring bilden,

$R^3$ und $R^4$ unter Einbezug der sie verbindenden Doppelbindung gemeinsam einen Phenylring oder einen 4- bis 8-gliedrigen Cycloalken-Rest bilden,
wobei alle unter $R^1/R^2$ und $R^3/R^4$ aufgeführten Ringsysteme gegebenenfalls bis zu 3-fach gleich oder verschieden durch Halogen, Trifluormethyl, Carboxy, Hydroxy, geradkettiges oder verzweigtes Alkoxy oder Alkoxycarbonyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy oder durch geradkettiges oder verzweigtes Alkoxy mit bis zu 4 Kohlenstoffatomen substituiert sein kann,

$R^5$ für Wasserstoff, Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano oder Carboxy steht,

D für Wasserstoff, Cycloalkyl mit 4 bis 8 Kohlenstoffatomen oder für geradkettiges oder verzweigtes Alkyl mit bis zu 8 Kohlenstoffatomen steht,

E für Wasserstoff oder für einen Rest der Formel
$-CO-NR^6R^7$,

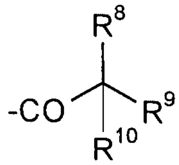
, $L-R^{11}$ oder
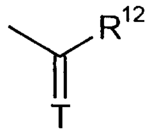

steht,
in welchen

$R^6$ und $R^7$ gleich oder verschieden sind und Wasserstoff, Phenyl oder geradkettiges oder verzweigtes Alkyl mit bis zu 6 Kohlenstoffatomen bedeuten,

$R^8$ Wasserstoff, geradkettiges oder verzweigtes Alkyl mit bis zu 6 Kohlenstoffatomen, Phenyl oder einen 5- bis 7-gliedrigen gesättigten oder ungesättigten Heterocyclus mit bis zu 3 Heteroatomen aus der Reihe S, N und/oder O bedeutet,
wobei die Cyclen gegebenenfalls bis zu 3-fach gleich oder verschieden durch Nitro, Carboxyl, Halogen, Cyano oder durch geradkettiges oder verzweigtes Alkoxycarbonyl mit bis zu 6 Kohlenstoffatomen oder durch geradkettiges oder verzweigtes Alkyl mit bis zu 6 Kohlenstoffatomen substituiert sind, das gegebenenfalls durch Hydroxy, Carboxyl oder durch geradkettiges oder verzweigtes Alkoxy oder Alkoxycarbonyl mit jeweils bis zu 6 Kohlenstoffatomen substituiert ist,
und/oder die Cyclen gegebenenfalls durch eine Gruppe der Formel $-OR^{13}$ substituiert sind,
worin

$R^{13}$ für Wasserstoff oder geradkettiges oder verzweigtes Alkyl oder Alkenyl mit jeweils bis zu 6 Kohlenstoffatomen steht,

$R^9$ Carboxyl, Hydroxy, geradkettiges oder verzweigtes Alkoxycarbonyl mit bis zu 6 Kohlenstoffatomen oder eine Gruppe der Formel $-CH_2OH-$ oder $-O-CO-R^{14}$ bedeutet,
worin

$R^{14}$ für geradkettiges oder verzweigtes Alkyl mit bis zu 6 Kohlenstoffatomen steht,

$R^{10}$ Wasserstoff bedeutet, oder

$R^9$ und $R^{10}$ gemeinsam einen Rest der Formel =O oder =N-$R^{15}$ bilden, worin

$R^{15}$ für Hydroxy, geradkettiges oder verzweigtes Alkoxy mit bis zu 6 Kohlenstoffatomen oder einen Rest der Formel $-NH-CO-NH_2$ oder $-NH-CS-NH_2$ steht,

L eine Gruppe $-SO_2-$ oder -CO- bedeutet,

$R^{11}$ Phenyl oder einen 5- bis 7-gliedrigen aromatischen Heterocyclus mit bis zu 3 Heteroatomen aus der Reihe S, N und/oder O bedeutet, oder geradkettiges oder verzweigtes Alkyl mit bis zu 6 Kohlenstoffatomen bedeutet, das gegebenenfalls durch Phenyl oder durch einen 5- bis 7-gliedrigen aromatischen Heterocyclus mit bis zu 3 Heteroatomen aus der Reihe S, N und/oder O substituiert ist, wobei alle Ringsysteme gegebenenfalls bis zu 3-fach gleich oder verschieden durch Hydroxy, Phenoxy, Halogen oder durch geradkettiges oder verzweigtes Alkyl oder Alkoxy mit jeweils bis zu 5 Kohlenstoffatomen substituiert sind,

T ein Sauerstoff- oder Schwefelatom bedeutet,

$R^{12}$ Phenoxy, Phenylthio oder geradkettiges oder verzweigtes Alkoxy mit bis zu 6 Kohlenstoffatomen bedeutet,

und deren Salze.

**2.** Amide und Sulfonamide von heterocyclisch substituierten Benzylaminen der Formel nach Anspruch 1 in welcher

$R^1$ und $R^2$ unter Einbezug der sie verbindenden Doppelbindung gemeinsam einen Phenyl- oder Pyridylring bilden,

$R^3$ und $R^4$ unter Einbezug der sie verbindenden Doppelbindung gemeinsam einen Phenylring oder einen Cyclopenten-, Cyclohexen-, Cyclohepten-, Cycloocten-Rest bilden, wobei alle unter $R^1$/$R^2$ und $R^3$/$R^4$ aufgeführten Ringsysteme gegebenenfalls bis zu 2-fach gleich oder verschieden durch Fluor, Chlor, Brom, Trifluormethyl, Carboxy, Hydroxy, geradkettiges oder verzweigtes Alkoxy oder Alkoxycarbonyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy oder durch geradkettiges oder verzweigtes Alkoxy mit bis zu 3 Kohlenstoffatomen substituiert sein kann,

$R^5$ für Wasserstoff, Fluor, Chlor, Brom, Trifluormethyl, Carboxy oder geradkettiges oder verzweigtes Alkyl, Alkoxy oder Alkoxycarbonyl mit jeweils bis zu 4 Kohlenstoffatomen steht,

D für Wasserstoff, Cyclobutyl, Cyclopentyl, Cyclohexyl, Cycloheptyl, Cylcooctyl oder für geradkettiges oder verzweigtes Alkyl mit bis zu 7 Kohlenstoffatomen steht,

E für Wasserstoff oder für einen Rest der Formel $-CO-NR^6R^7$,

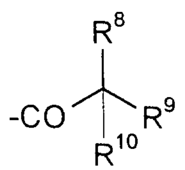 , $L-R^{11}$ oder 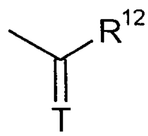

steht,

in welchen

$R^6$ und $R^7$ gleich oder verschieden sind und Wasserstoff, Phenyl oder geradkettiges oder verzweigtes Alkyl mit bis zu 6 Kohlenstoffatomen bedeuten,

$R^8$ Wasserstoff, geradkettiges oder verzweigtes Alkyl mit bis zu 6 Kohlenstoffatomen, Phenyl, Pyridyl, Furyl, Thienyl oder Imidazolyl bedeutet, die gegebenenfalls bis zu 2-fach gleich oder verschieden durch Nitro, Carboxyl, Fluor, Chlor, Brom, Cyano, durch geradkettiges oder verzweigtes Alkoxycarbonyl mit bis zu 4 Kohlenstoffatomen oder durch geradkettiges oder verzweigtes Alkyl mit bis zu 5 Kohlenstoffatomen substituiert sind, das gegebenenfalls durch Hydroxy, Carboxyl oder durch geradkettiges oder verzweigtes Alkoxy oder Alkoxycarbonyl mit jeweils bis zu 5 Kohlenstoffatomen substituiert ist,
und/oder die Cyclen gegebenenfalls durch eine Gruppe der Formel $-OR^{13}$ substituiert sind, worin

$R^{13}$ für Wasserstoff oder geradkettiges oder verzweigtes Alkyl oder Alkenyl mit jeweils bis zu 4 Kohlenstoffatomen steht,

$R^9$ Carboxyl, Hydroxy, geradkettiges oder verzweigtes Alkoxycarbonyl mit bis zu 4 Kohlenstoffatomen oder eine Gruppe der Formel $-CH_2OH$ oder $-O-CO-R^{14}$ bedeutet, worin

$R^{14}$ für geradkettiges oder verzweigtes Alkyl mit bis zu 4 Kohlenstoffatomen steht,

$R^{10}$ Wasserstoff bedeutet,
oder

$R^9$ und $R^{10}$ gemeinsam einen Rest der Formel =O oder $=N-R^{15}$ bilden, worin

$R^{15}$ für Hydroxy, geradkettiges oder verzweigtes Alkoxy mit bis zu 4 Kohlenstoffatomen oder einen Rest der Formel $-NH-CO-NH_2$ oder $-NH-CS-NH_2$ steht,

L eine Gruppe $-SO_2-$ oder -CO- bedeutet,

$R^{11}$ Phenyl, Pyridyl, Thienyl, Furyl oder Pyrimidyl oder geradkettiges oder verzweigtes Alkyl mit bis zu 4 Kohlenstoffatomen bedeutet, das gegebenenfalls durch Pyridyl, Thienyl, Furyl oder Pyrimidyl substituiert ist, wobei alle Ringsysteme gegebenenfalls bis zu 2-fach gleich oder verschieden durch Hydroxy, Phenoxy, Fluor, Chlor, Brom oder durch geradkettiges oder verzweigtes Alkyl oder Alkoxy mit jeweils bis zu 4 Kohlenstoffatomen substituiert sind,

T ein Schwefel- oder Sauerstoffatom bedeutet,

$R^{12}$ Phenoxy, Phenylthio oder geradkettiges oder verzweigtes Alkoxy mit bis zu 4 Kohlenstoffatomen bedeutet

und deren Salze.

**3.** Amide und Sulfonamide von heterocyclisch substituierten Benzylaminen der Formel nach Anspruch 1 in welcher

$R^1$ und $R^2$ unter Einbezug der sie verbindenden Doppelbindung gemeinsam einen Phenyl- oder Pyridylring bilden,

$R^3$ und $R^4$ unter Einbezug der sie verbindenden Doppelbindrung gemeinsam einen Phenylring oder einen Cyclopenten-, Cyclohexen-, Cyclohepten-, Cycloocten-Rest bilden,
wobei alle unter $R^1/R^2$ und $R^3/R^4$ aufgeführten Ringsysteme gegebenenfalls bis zu 2-fach gleich oder verschieden durch Fluor, Chlor, Brom, Trifluormethyl, Carboxy, Hydroxy, durch geradkettiges oder verzweigtes Alkoxy oder Alkoxycarbonyl mit jeweils bis zu 3 Kohlenstoffatomen oder durch geradkettiges oder verzweigtes Alkyl mit bis zu 3 Kohlenstoffatomen substituiert sind, das seinerseits durch Hydroxy, Methoxy oder Ethoxy substituiert sein kann,

$R^5$ für Wasserstoff, Fluor, Chlor, Brom, Trifluormethyl, Trifluormethoxy oder Methyl steht,

D für Wasserstoff, Cyclopentyl, Cyclohexyl, Cycloheptyl, Cylcooctyl oder für geradkettiges oder verzweigtes Alkyl mit bis zu 6 Kohlenstoffatomen steht,

E für Wasserstoff oder für einen Rest der Formel
$-CO-NR^6R^7$,

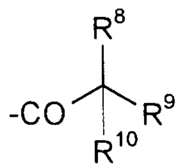
, L-$R^{11}$ oder
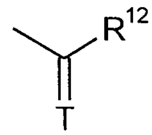

steht,
in welchen

$R^6$ und $R^7$ gleich oder verschieden sind und Wasserstoff, Phenyl oder geradkettiges oder verzweigtes Alkyl mit bis zu 6 Kohlenstoffatomen bedeuten,

$R^8$ Wasserstoff, geradkettiges oder verzweigtes Alkyl mit bis zu 6 Kohlenstoffatomen, Phenyl, Pyridyl oder Thienyl bedeutet, die gegebenenfalls bis zu 2-fach gleich oder verschieden durch Nitro, Carboxyl, Fluor, Chlor, Brom, Cyano, durch geradkettiges oder verzweigtes Alkoxycarbonyl mit bis zu 3 Kohlenstoffatomen oder durch geradkettiges oder verzweigtes Alkyl mit bis zu 4 Kohlenstoffatomen substituiert sind, das gegebenenfalls durch Hydroxy, Carboxyl oder durch geradkettiges oder verzweigtes Alkoxy oder Alkoxycarbonyl mit jeweils bis zu 4 Kohlenstoffatomen substituiert ist,
und/oder die Cyclen gegebenenfalls durch eine Gruppe der Formel $-OR^{13}$ substituiert sind,
worin

$R^{13}$ für Wasserstoff oder geradkettiges oder verzweigtes Alkyl oder Alkenyl mit jeweils bis zu 3 Kohlenstoffatomen steht,

$R^9$ Carboxyl, Hydroxy, geradkettiges oder verzweigtes Alkoxycarbonyl mit bis zu 3 Kohlenstoffatomen oder eine Gruppe der Formel $-CH_2OH$ oder $-O-CO-R^{14}$ bedeutet,
in welcher

$R^{14}$ für geradkettiges oder verzweigtes Alkyl mit bis zu 3 Kohlenstoffatomen steht,

$R^{10}$ Wasserstoff bedeutet,
oder

$R^9$ und $R^{10}$ gemeinsam einen Rest der Formel =O oder $=N-R^{15}$ bilden,
worin

$R^{15}$ Hydroxy, geradkettiges oder verzweigtes Alkoxy mit bis zu 3 Kohlenstoffatomen oder einen Rest der Formel $-NH-CO-NH_2$ oder $-NH-CS-NH_2$ bedeutet,

L einen Rest der Formel $-SO_2$ oder -CO bedeutet,

$R^{11}$ Phenyl, Pyridyl oder Thienyl oder geradkettiges oder verzweigtes Alkyl mit bis zu 3 Kohlenstoffatomen bedeutet, das gegebenenfalls durch Phenyl, Pyridyl oder Thienyl substituiert ist, wobei alle Ringsysteme gegebenenfalls bis zu 2-fach gleich oder verschieden durch Hydroxy, Phenoxy, Fluor, Chlor, Brom oder durch geradkettiges oder verzweigtes Alkyl oder

Alkoxy mit jeweils bis zu 3 Kohlenstoffatomen substituiert sind,

T ein Schwefel- oder Sauerstoffatom bedeutet,

$R^{12}$ Phenoxy, Phenylthio oder geradkettiges oder verzweigtes Alkoxy mit bis zu 3 Kohlenstoffatomen bedeutet,

und deren Salze.

**4.** Amide und Sulfonamide von heterocyclisch substituierten Benzylaminen nach Anspruch 1 bis 3 zur therapeutischen Verwendung.

**5.** Verfahren zur Herstellung von Amiden und Sulfonamiden von heterocyclisch substituierten Benzylaminen nach Anspruch 1 bis 3, dadurch gekennzeichnet, daß man Carbonsäuren der allgemeinen Formel (II)

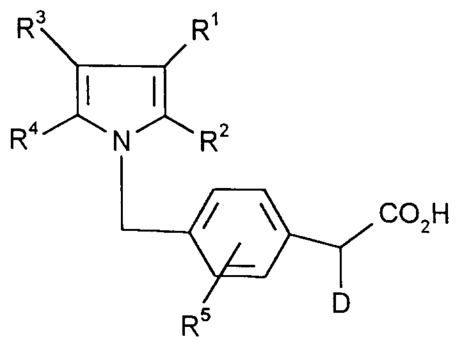

(II)

in welcher
$R^1$, $R^2$, $R^3$, $R^4$, $R^5$ und D die angegebene Bedeutung haben, zunächst durch Umsetzung mit Phosphorsäurediphenylesterazid in inerten Lösemitteln und in Anwesenheit einer Base in die Amine der allgemeinen Formel (Ia)

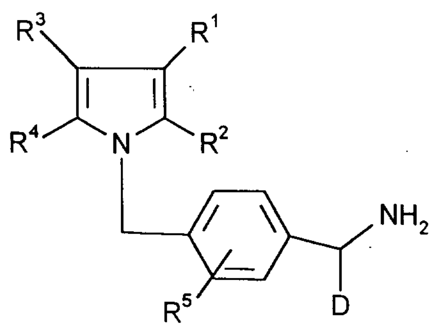

(Ia)

in welcher
$R^1$, $R^2$, $R^3$, $R^4$, $R^5$ und D die angegebene Bedeutung haben,
überführt,
und anschließend mit Verbindungen der allgemeinen Formel (III)

$$E^1\text{-}X \qquad \text{(III)}$$

in welcher

$E^1$ die oben angegebene Bedeutung von E hat aber nicht für Wasserstoff steht,
und

X in Abhängigkeit von den unter E angegebenen Resten für Hydroxy oder Halogen steht,

in inerten organischen Lösemitteln, gegebenenfalls in Anwesenheit einer Base und/oder von Hilfsmitteln umsetzt, und gegebenenfalls derivatisiert oder variiert.

**6.** Arzneimittel enthaltend mindestens ein Amid oder Sulfonamid von heterocyclisch substituierten Benzylaminen nach Anspruch 1 bis 3.

**7.** Arzneimittel nach Anspruch 6 zur Behandlung von Atherosklerose.

**8.** Verwendung von Amiden und Sulfonamiden von heterocyclisch substituierten Benzylaminen nach Anspruch 1 bis 3 zur Herstellung von Arzneimitteln.

**9.** Carbonsäuren der allgemeinen Formel (II)

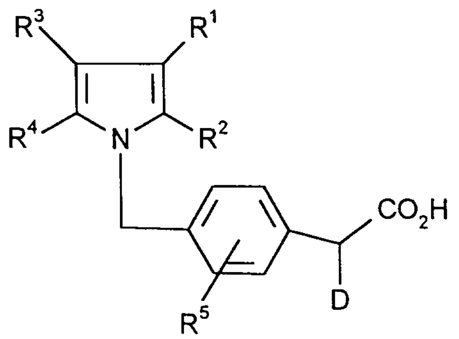

(II)

in welcher

$R^1$ und $R^2$ unter Einbezug der sie verbindenden Doppelbindung gemeinsam einen Phenyl- oder Pyridylring bilden,

$R^3$ und $R^4$ unter Einbezug der sie verbindenden Doppelbindung gemeinsam einen Phenylring oder einen 4- bis 8-gliedrigen Cycloalken-Rest bilden,
wobei alle unter $R^1$/$R^2$ und $R^3$/$R^4$ aufgeführten Ringsysteme gegebenenfalls bis zu 3-fach gleich oder verschieden durch Halogen, Trifluormethyl, Carboxy, Hydroxy, geradkettiges oder verzweigtes Alkoxy oder Alkoxycarbonyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy oder durch geradkettiges oder verzweigtes Alkoxy mit bis zu 4 Kohlenstoffatomen substituiert sein kann,

$R^5$ für Wasserstoff, Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano oder Carboxy steht,

D für Wasserstoff, Cycloalkyl mit 4 bis 8 Kohlenstoffatomen oder für geradkettiges oder verzweigtes Alkyl mit bis zu 8 Kohlenstoffatomen steht.

**10.** Verfahren zur Herstellung von Carbonsäuren nach Anspruch 9, dadurch gekennzeichnet, daß man Verbindungen der allgemeinen Formel (IV)

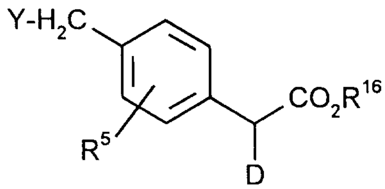

(IV)

in welcher

D und $R^5$ die angegebene Bedeutung haben,

Y für eine typische Abgangsgruppe wie beispielsweise Chlor, Brom, Jod, Tosylat oder Mesylat, vorzugsweise für Brom steht,
und

$R^{16}$ für geradkettiges oder verzweigtes Alkyl mit bis zu 4 Kohlenstoffatomen steht,
mit Verbindungen der allgemeinen Formel (V)

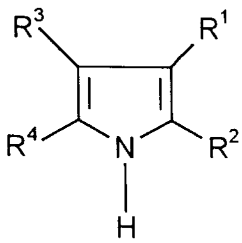

(V)

in welcher
$R^1$, $R^2$, $R^3$ und $R^4$ die angegebene Bedeutung haben
in inerten Lösemitteln, gegebenenfalls in Anwesenheit einer Base, umsetzt.